# EUROPEAN PATENT SPECIFICATION

(11) **EP 2 663 609 B1**
(45) Date of publication and mention of the grant of the patent: **06.01.2016**
(21) Application number: 11866266.7
(22) Date of filing: 04.10.2011
(51) Int. Cl.: C09K 11/06, H01L 51/54, H01L 51/52

(54) **COMPOUND FOR ORGANIC OPTOELECTRONIC DEVICE, ORGANIC LIGHT EMITTING DIODE INCLUDING THE SAME AND DISPLAY INCLUDING THE ORGANIC LIGHT EMITTING DIODE**
VERBINDUNG FÜR EINE ORGANISCHE OPTOELEKTRONISCHE VORRICHTUNG, ORGANISCHE LICHTEMITTIERENDE DIODE DAMIT UND ANZEIGE MIT DER ORGANISCHEN LICHTEMITTIERENDEN DIODE
COMPOSÉ POUR DISPOSITIF OPTOÉLECTRONIQUE ORGANIQUE, DIODE ÉLECTROLUMINESCENTE ORGANIQUE LE COMPRENANT ET AFFICHEUR COMPRENANT LA DIODE ÉLECTROLUMINESCENTE ORGANIQUE

(30) Priority: 26.05.2011 KR 20110050344
(43) Date of publication of application: 20.11.2013
(73) Proprietor: Cheil Industries Inc., Kumi-city, Kyungsangbuk-do 730-030 (KR)
(72) Inventor: YU, Eun-Sun, Uiwang-si Gyeonggi-do 437-711 (KR); CHAE, Mi-Young, Uiwang-si Gyeonggi-do 437-711 (KR); KANG, Eui-Su, Uiwang-si Gyeonggi-do 437-711 (KR); LEE, Nam-Heon, Uiwang-si Gyeonggi-do 437-711 (KR)
(74) Representative: Michalski, Stefan
(86) International application number: PCT/KR2011/007315
(87) International publication number: WO 2012/161382

(56) References cited:
- WO-A1-2006/025186
- WO-A1-2011/019156
- WO-A1-2012/023947
- WO-A1-2012/087007
- WO-A1-2012/108881
- WO-A2-2011/055934
- KR-A- 20110 122 051

## Description

### Technical Field

A compound for an organic optoelectronic device that is capable of providing an organic optoelectronic device having excellent life-span, efficiency, electrochemical stability, and thermal stability, an organic light emitting diode and a display device including the organic light emitting diode are related.

### Background Art

An organic optoelectric device is a device requiring a charge exchange between an electrode and an organic material by using a hole or an electron.

WO 2012/108881 A1 refers to OLEDs with an organic thin film layer comprising a single layer or plural layers between a cathode and an anode, wherein the organic thin film layer comprises at least one organic light emitting layer, wherein at least one light emitting layer comprises at least one host material and at least one phosphorescent emitter material, wherein the host material comprises a bis-carbazole derivative host material; and the phosphorescent emitter material comprises a phosphorescent organometallic complex having a substituted chemical structure represented by one of the following partial chemical structures represented by the formula: LL'L"M wherein M is a metal that forms octahedral complexes, L, L', L" are equivalent or inequivalent bidentate ligands wherein each L comprises a substituted or unsubstituted phenylpyridine ligand coordinated to M through an sp2 hybridized carbon and N; and one of L, L' and L" is inequivalent to at least one of the other two.

WO 2012/087007 A1 refers to organic electroluminescent compounds and organic electroluminescent devices using the same. The organic electroluminescent compound exhibits good luminous efficiency and excellent life property, it may be used to manufacture OLED devices having superior operation life and consuming less power due to improved power efficiency.

WO 2011/055934 A2 refers to a compound for an organic photoelectric device and an organic photoelectric device including the same are disclosed, and the compound for an organic photoelectric device is represented by Chemical Formula 1.

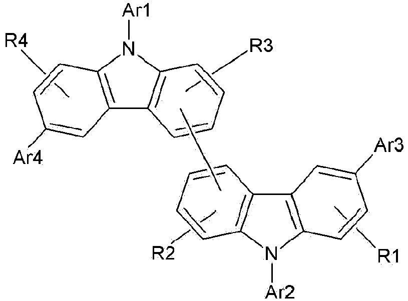

An organic optoelectric device may be classified as follows in accordance with its driving principles. A first organic optoelectric device is an electron device driven as follows: excitons are generated in an organic material layer by photons from an external light source; the excitons are separated into electrons and holes; and the electrons and holes are transferred to different electrodes as a current source (voltage source).

A second organic optoelectric device is an electron device driven as follows: a voltage or a current is applied to at least two electrodes to inject holes and/or electrons into an organic material semiconductor positioned at an interface of the electrodes; and the device is driven by the injected electrons and holes.

As examples, the organic optoelectric device includes an organic light emitting diode (OLED), an organic solar cell, an organic photo-conductor drum, an organic transistor, an organic memory device, etc., and it requires a hole injecting or transporting material, an electron injecting or transporting material, or a light emitting material.

Particularly, the organic light emitting diode (OLED) has recently drawn attention due to an increase in demand for flat panel displays. In general, organic light emission refers to transformation of electrical energy to photo-energy.

The organic light emitting diode transforms electrical energy into light by applying current to an organic light emitting material. It has a structure in which a functional organic material layer is interposed between an anode and a cathode. The organic material layer includes a multi-layer including different materials, for example a hole injection layer (HIL), a hole transport layer (HTL), an emission layer, an electron transport layer (ETL), and an electron injection layer (EIL), in order to improve efficiency and stability of an organic light emitting diode.

In such an organic light emitting diode, when a voltage is applied between an anode and a cathode, holes from the anode and electrons from the cathode are injected to an organic material layer. The generated excitons generate light having certain wavelengths while shifting to a ground state.

Recently, it has become known that a phosphorescent light emitting material can be used for a light emitting material of an organic light emitting diode in addition to the fluorescent light emitting material. Such a phosphorescent material emits lights by transiting the electrons from a ground state to an exited state, non-radiance transiting of a singlet exciton to a triplet exciton through intersystem crossing, and transiting a triplet exciton to a ground state to emit light.

As described above, in an organic light emitting diode, an organic material layer includes a light emitting material and a charge transport material, for example a hole injection material, a hole transport material, an electron transport material, an electron injection material, and so on.

The light emitting material is classified as blue, green, and red light emitting materials according to emitted colors, and yellow and orange light emitting materials to emit colors approaching natural colors.

When one material is used as a light emitting material, a maximum light emitting wavelength is shifted to a long wavelength or color purity decreases because of interactions between molecules, or device efficiency decreases because of a light emitting quenching effect. Therefore, a host/dopant system is included as a light emitting material in order to improve color purity and increase luminous efficiency and stability through energy transfer.

In order to implement excellent performance of an organic light emitting diode, a material constituting an organic material layer, for example a hole injection material, a hole transport material, a light emitting material, an electron transport material, an electron injection material, and a light emitting material such as a host and/or a dopant, should be stable and have good efficiency. However, development of an organic material layer forming material for an organic light emitting diode has thus far not been satisfactory and thus there is a need for a novel material. This material development is also required for other organic optoelectric devices.

The low molecular organic light emitting diode is manufactured as a thin film in a vacuum deposition method and can have good efficiency and life-span performance. A polymer organic light emitting diode is manufactured in an Inkjet or spin coating method has an advantage of low initial cost and being large-sized. Recently, a low molecular material using a solution process shows better performance than a polymer material, and thus developments for a low molecular material has been concentrated.

Both low molecular organic light emitting and polymer organic light emitting diodes have an advantage of self-light emitting, high speed response, wide viewing angle, ultrathin, high image quality, durability, large driving temperature range, and the like. In particular, they have good visibility due to self-light emitting characteristic compared with a conventional LCD (liquid crystal display) and have an advantage of decreasing thickness and weight of LCD up to a third, because they do not need a backlight.

In addition, since they have a response speed 1000 time faster microsecond unit than LCD, they can realize a perfect motion picture without after-image. Based on these advantages, they have been remarkably developed to have 80 times efficiency and more than 100 times life-span since they come out for the first time in the late 1980s. Recently, they keep being rapidly larger such as a 40-inch organic light emitting diode panel.

They are simultaneously required to have improved luminous efficiency and life-span in order to be larger. Herein, their luminous efficiency need smooth combination between holes and electrons in an emission layer. However, since an organic material in general has slower electron mobility than hole mobility, it has a drawback of inefficient combination between holes and electrons. Accordingly, while increasing electron injection and mobility from a cathode and simultaneously preventing movement of holes is required.

In order to improve life-span, a material crystallization caused by Joule heats generated during device operating is required to be prevented. Accordingly, there has been a strong need for an organic compound having excellent electron injection and mobility, and high electrochemical stability.

### DISCLOSURE

### Technical Problem

A compound for an organic optoelectronic device that may act as a hole injection and hole transport, or an electron injection and transport, and also act as a light emitting host along with an appropriate dopant is provided.

### Technical Solution

A light emitting diode having excellent life span, efficiency, a driving voltage, electrochemical stability, and thermal stability and a display device including the same are provided.

According to one embodiment of the present invention, a compound for an organic optoelectronic device represented by the following Chemical Formula 1 is provided.

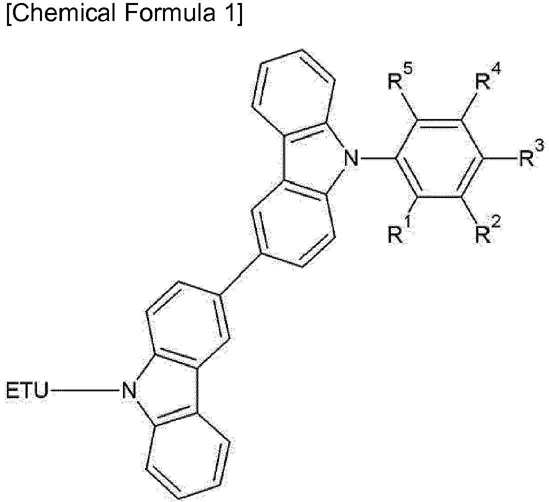

In Chemical Formula 1, ETU is a substituted or unsubstituted pyridinyl group, a substituted or unsubstituted pyrimidinyl group, a substituted or unsubstituted triazinyl group, or a combination thereof, provided at least one of R¹ to R⁵ is a substituted or unsubstituted C1 to C30 alkyl group.

At least one of R² or R⁴ may be a substituted or unsubstituted C1 to C30 alkyl group.

At least one of R² to R⁴ may be a substituted or unsubstituted methyl group.

The ETU may be a substituent represented by the following Chemical Formulas 2 to 6.

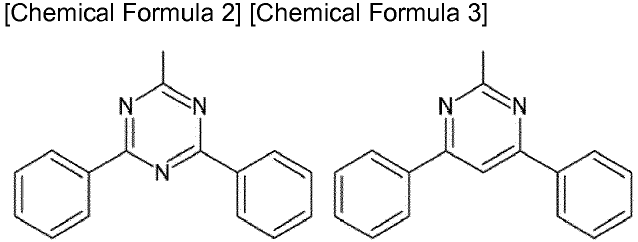

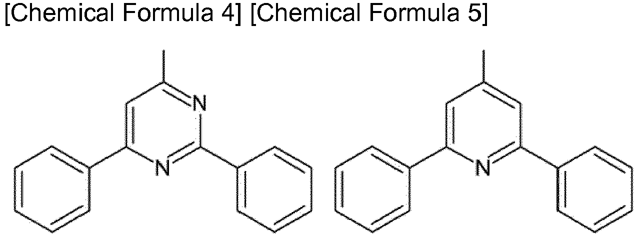

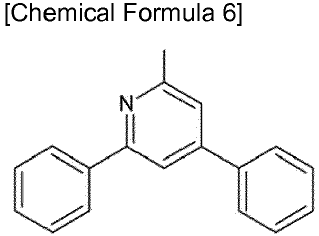

The compound for an organic optoelectronic device may be represented by the following Chemical Formulas B-1 to B-25.

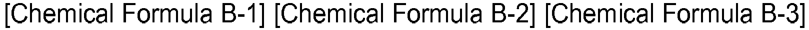

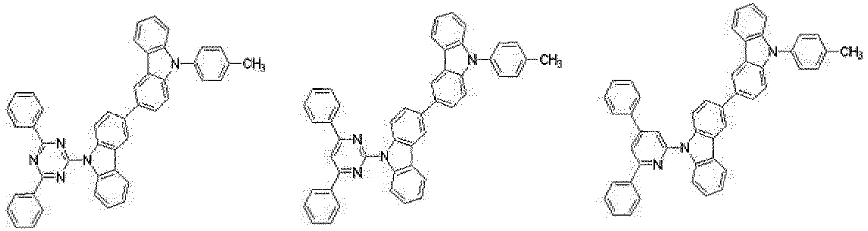

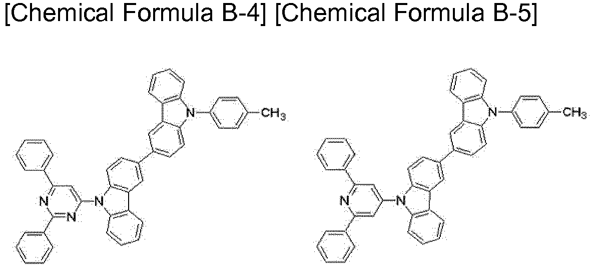

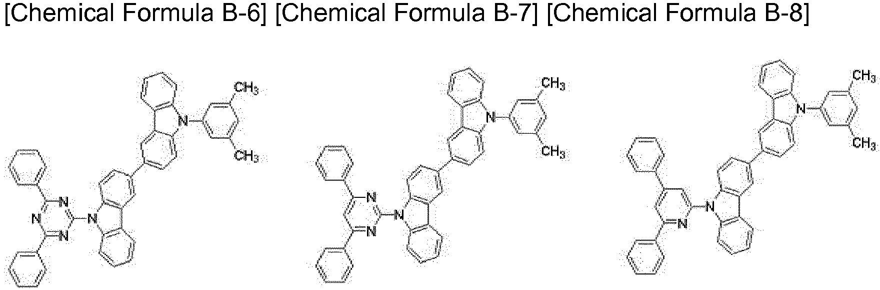

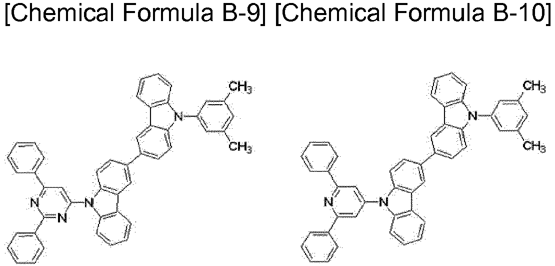

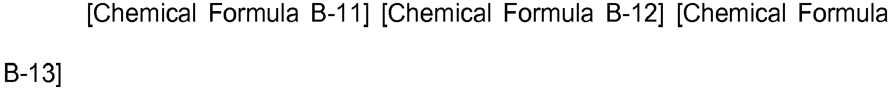

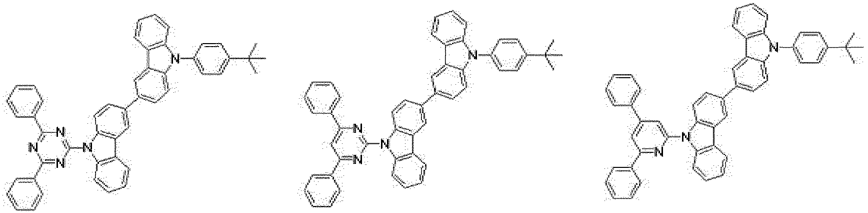

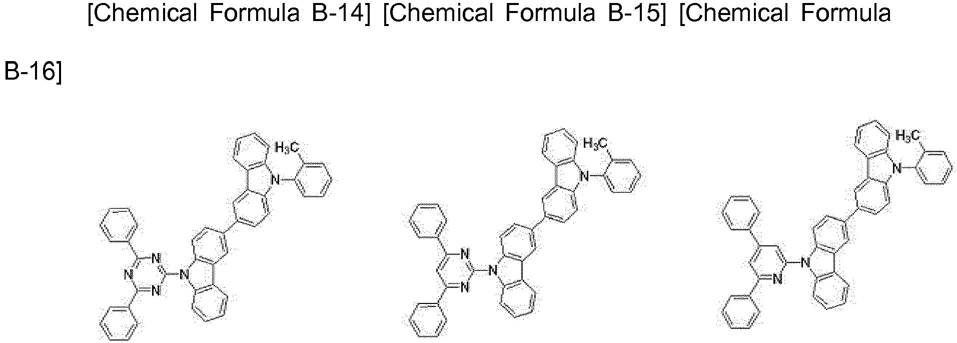

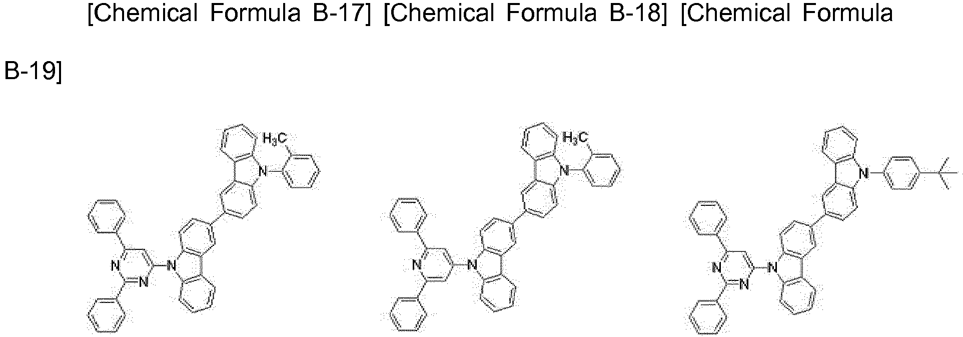

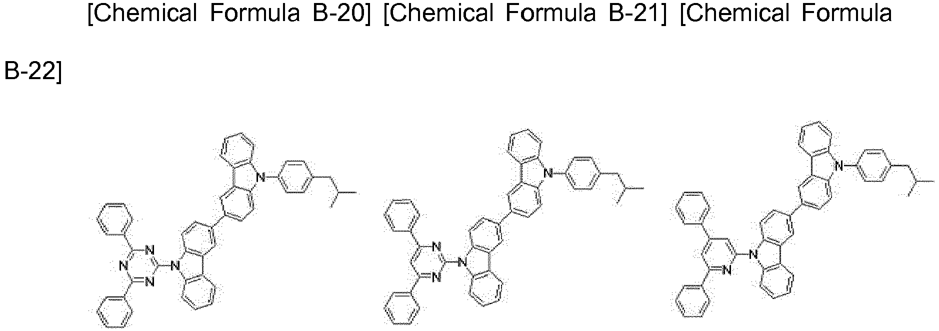

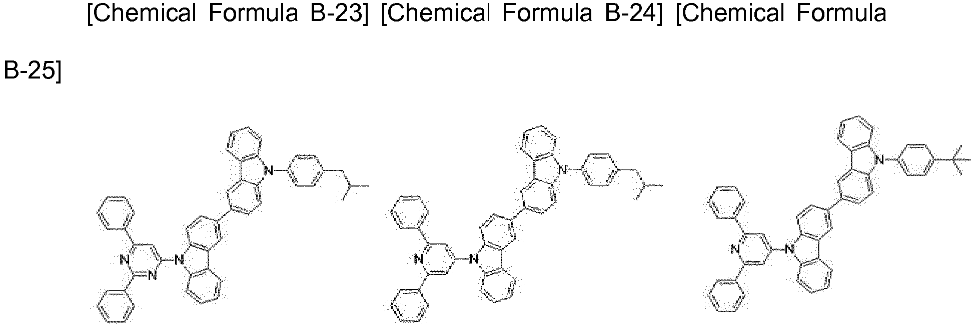

The compound for an organic optoelectronic device may have a triplet excitation energy (T1) of 2.0eV or more.

The organic optoelectronic device may be selected from an organic photoelectric device, an organic light emitting diode, an organic solar cell, an organic transistor, an organic photo-conductor drum, and an organic memory device.

According to another embodiment of the present invention, provided is an organic light emitting diode that includes an anode, a cathode, and at least one or more organic thin layer between the anode and the cathode, wherein at least one of the organic thin layer includes the compound for an organic optoelectronic device.

The organic thin layer may include an emission layer, a hole transport layer (HTL), a hole injection layer (HIL), an electron transport layer (ETL), an electron injection layer (EIL), a hole blocking layer, or a combination thereof.

The compound for an organic optoelectronic device may be included in a hole transport layer (HTL) or a hole injection layer (HIL).

The compound for an organic optoelectronic device may be included in an emission layer.

The compound for an organic optoelectronic device may be used as a phosphorescent or fluorescent host material in an emission layer.

According to another embodiment of the present invention, a display device including the organic light emitting diode is provided.

### Advantageous Effects

The compound for an organic optoelectronic device has an excellent hole or electron transporting property, high film stability, thermal stability, and triplet excitation energy.

The compound may be used as a hole injection/ transport material of an emission layer, a host material, or an electron injection/ transport material. The organic optoelectronic device has an excellent electrochemical and thermal stability, and therefore, may provide an organic light emitting diode having an excellent life-span characteristic, and high luminous efficiency at a low driving voltage.

### Description of the Drawings

FIGS. 1 to 5 are cross-sectional views showing organic light emitting diodes including compounds according to various embodiments of the present invention.
FIG. 6 shows life-span data of the organic light emitting diodes according to Examples not according to the invention, Example 11 and Comparative Example 4.
FIG. 7 shows life-span data of the organic light emitting diodes according to an Example not according to the invention, Example 12 and Comparative Example 5.
FIG. 8 shows life-span data of the organic light emitting diodes according to an Example not according to the invention and Comparative Example 6.

### Mode for Invention

Exemplary embodiments of the present invention will hereinafter be described in detail. However, these embodiments are only exemplary, and the present invention is not limited thereto but rather is defined by the scope of the appended claims.

As used herein, when specific definition is not otherwise provided, the term "substituted" refers to one substituted with deuterium, a halogen, a hydroxy group, an amino group, a substituted or unsubstituted C1 to C20 amine group, a nitro group, a substituted or unsubstituted C3 to C40 silyl group, a C1 to C30 alkyl group, a C1 to C10 alkylsilyl group, a C3 to C30 cyclo alkyl group, a C6 to C30 aryl group, a C1 to C20 alkoxy group, a fluoro group, a C1 to C10 trifluoro alkyl group such as a trifluoromethyl group, or a cyano group, instead of hydrogen.

Two adjacent substituents of the substituted a hydroxy group, amino group, a substituted or unsubstituted C1 to C20 amine group, nitro group, a substituted or unsubstituted C3 to C40 silyl group, a C1 to C30 alkyl group, a C1 to C10 an alkylsilyl group, C3 to C30 cycloalkyl group, a C6 to C30 aryl group, C1 to C20 alkoxy group, a C1 to C10 trifluoroalkyl group such as a trifluoromethyl group, or a cyano group are linked to each other to provide a fused ring.

As used herein, when specific definition is not otherwise provided, the term "hetero" refers to one including 1 to 3 of N, O, S, or P, and remaining carbons in one ring.

As used herein, when a definition is not otherwise provided, the term "combination thereof" refers to at least two substituents bound to each other by a linker, or at least two substituents condensed to each other.

As used herein, when a definition is not otherwise provided, the term "alkyl group" refers to an aliphatic hydrocarbon group. The alkyl may be a saturated alkyl group that does not include any double bond or triple bond.

Alternatively, the alkyl may be an unsaturated alkyl group that includes at least one double bond or triple bond.

The term "alkenylene group" may refer to a group in which at least two carbon atoms are bound in at least one carbon-carbon double bond, and the term "alkynylene group" may refer to a group in which at least two carbon atoms are bound in at least one carbon-carbon triple bond. Regardless of being saturated or unsaturated, the alkyl may be branched, linear, or cyclic.

The alkyl group may be a C1 to C20 alkyl group. More particularly, the alkyl group may be a C1 to C10 alkyl group or a C1 to C6 alkyl group.

For example, a C1 to C4 alkyl group may have 1 to 4 carbon atoms and may be selected from the group consisting of methyl, ethyl, propyl, iso-propyl, n-butyl, iso-butyl, sec-butyl, and t-butyl.

Examples of the alkyl group may be a methyl group, an ethyl group, a propyl group, an isopropyl group, a butyl group, an isobutyl group, a t-butyl group, a pentyl group, a hexyl group, an ethenyl group, a propenyl group, a butenyl group, a cyclopropyl group, a cyclobutyl group, a cyclopentyl group, a cyclohexyl group, and the like.

The term "aromatic group" may refer to a cyclic functional group where all elements have conjugated p-orbital. Examples of the aromatic group include an aryl group and a heteroaryl group.

The term "aryl group" may refer to an aryl group including a carbocyclic aryl (e.g., phenyl) having at least one ring having a covalent pi electron system.

The term "heteroaryl group" may refer to an aryl group where 1 to 3 heteroatoms selected from N, O, S, and P, and remaining carbon. When the heteroaryl group is a fused ring, each ring may include 1 to 3 heteroatoms.

As used herein, a carbazole-based derivative indicates a substituted or unsubstituted carbazolyl group including another hetero atom substituted for a nitrogen atom. In particular, the substituted or unsubstituted carbazolyl group may include a dibenzofuranyl group, a dibenzothiophenyl group, and the like.

As used herein, hole properties refer to that holes generated at an anode are easily injected into an emission layer and moved therein due to conduction properties according to HOMO levels.

Electron properties refer to that electrons generated at a cathode are easily injected into an emission layer and moved therein due to conduction properties according to LUMO levels.

According to one embodiment of the present invention, a compound for an organic optoelectronic device may have a core including two carbazolyl groups and a phenyl group bonded with either one of the two carbazolyl group.

In addition, the phenyl group in the core may be bonded with at least one substituted or unsubstituted C1 to C30 alkyl group.

Furthermore, the core may include a substituent having electron properties.

The core structure includes a substituent having electron properties combined with a carbazolyl group having hole properties and thus, may be applied to a light emitting material, a hole injection material, or a hole transport material for an organic optoelectronic device. In particular, it may be applied to a light emitting material.

In addition, at least one substituted or unsubstituted C1 to C30 alkyl group in the core may decrease molecular interaction and thus, lower crystallinity of the compound when the compound for an organic optoelectronic device is used to form a layer. As a result, the compound in a device may be suppressed from recrystallization.

At least one substituent bonded to the core may have electron properties. Accordingly, the compound may be reinforced with electron properties as well as have a carbazole structure with excellent hole properties, which may satisfy conditions required of an emission layer. In particular, the compound may be used as a host material for an emission layer.

In addition, the compound for an organic optoelectronic device may have various energy band gaps by introducing another substituent to a core moiety and a substituent substituted in the core moiety.

When the compound having an appropriate energy level depending on a substituent is used for an organic optoelectronic device, the compound reinforce a hole transporting property or an electron transporting property of a layer, and the organic optoelectronic device may have excellent efficiency and a driving voltage. In addition, the compound has excellent electrochemical and thermal stability and thus, may improve life-span characteristic of the organic optoelectronic device.

In the compound for an organic optoelectronic device, at least one of R¹ to R⁵ may have a substituent selected from a substituted or unsubstituted C1 to C30 alkyl group and the substituent provides the compound for an organic optoelectronic device with light emitting, hole or electron properties; film stability; thermal stability, and high triplet excitation energy (T1).

In addition, a compound for an organic optoelectronic device including the substituent may not form a composite due to dipole-dipole strength among the molecules. When the composite is formed, HOMO/LUMO energy bandgap may be smaller than energy bandgap of a single molecule. When a compound easily forming the above composite is used for a device, the device may have decreased luminous efficiency and life-span.

In addition, a compound for an organic optoelectronic device including the substituent, the compound may not have a planar structure and less crystalline. However, a device fabricated by using a compound easily forming crystalline may be degraded during the repetitive operation and thus, decreased life-span.

In addition, a compound for an organic optoelectronic device including the substituent may have improved bulk characteristics. When the bulk characteristics of a compound are appropriately adjusted, a device may accomplish desired characteristics.

At least one of R² or R⁴ may be a substituted or unsubstituted C1 to C30 alkyl group.

At least one of R² to R⁴ may be a substituted or unsubstituted methyl group. However, at least one of R² to R⁴ is not limited thereto.

When the aforementioned compound according to one embodiment of the present invention is required of both electron properties and hole properties, the functional group with electron properties may be included in a compound to effectively improve life-span of an organic light emitting diode and decrease its driving voltage.

According to the embodiment of the present invention, the compound for an organic optoelectronic device has a maximum light emitting wavelength ranging from 320 to 500 nm and triplet excitation energy of 2.0 eV or more (T1), and specifically, ranging from 2.0 to 4.0 eV. When it has this high excitation energy, it can transport a charge to a dopant well and improve luminous efficiency of the dopant, and can also decrease the driving voltage by freely regulating HOMO and LUMO energy levels. Accordingly, it can be usefully applied as a host material or a charge-transporting material.

The compound for an organic optoelectronic device may be also used as a nonlinear optical material, an electrode material, a chromic material, and as a material applicable to an optical switch, a sensor, a module, a waveguide, an organic transistor, a laser, an optical absorber, a dielectric material, and a membrane due to its optical and electrical properties.

The compound for an organic optoelectronic device including the above compound has a glass transition temperature of 90°C or higher and a thermal decomposition temperature of 400°C or higher, so as to improve thermal stability. Thereby, it is possible to produce an organic optoelectronic device having a high efficiency.

The compound for an organic optoelectronic device including the above compound may play a role in emitting light or injecting and/or transporting electrons, and it may act as a light emitting host together with a suitable dopant. In other words, the compound for an organic optoelectronic device may be used as a phosphorescent or fluorescent host material, a blue light emitting dopant material, or an electron transporting material.

Since the compound for an organic optoelectronic device according to one embodiment is used for an organic thin layer, it may improve the life span characteristic, efficiency characteristic, electrochemical stability, and thermal stability of an organic optoelectronic device, and decrease the driving voltage.

Therefore, according to another embodiment, an organic optoelectronic device is provided that includes the compound for an organic optoelectronic device. The organic optoelectronic device may refer to an organic photoelectric device, an organic light emitting diode, an organic solar cell, an organic transistor, an organic photo-conductor drum, an organic memory device, and the like. For example, the compound for an organic optoelectronic device according to one embodiment may be included in an electrode or an electrode buffer layer in the organic solar cell to improve quantum efficiency, and it may be used as an electrode material for a gate, a source-drain electrode, or the like in the organic transistor.

Hereinafter, a detailed described relating to the organic light emitting diode will be provided.

According to another embodiment of the present invention, an organic light emitting diode includes an anode, a cathode, and at least one organic thin layer interposed between the anode and the cathode, wherein at least one organic thin layer may provide an organic optoelectronic device including the compound for an organic optoelectronic device according to one embodiment.

The organic thin layer that may include the compound for an organic optoelectronic device may include a layer selected from the group consisting of an emission layer, a hole transport layer (HTL), a hole injection layer (HIL), an electron transport layer (ETL), an electron injection layer (EIL), a hole blocking film, and a combination thereof. At least one layer includes the compound for an organic optoelectronic device according to one embodiment. Particularly, the electron transport layer (ETL) or the electron injection layer (EIL) may include the compound for an organic optoelectronic device according to one embodiment. In addition, when the compound for an organic photoelectric device is included in the emission layer, the compound for an organic photoelectric device may be included as a phosphorescent or fluorescent host, and particularly, as a fluorescent blue dopant material.

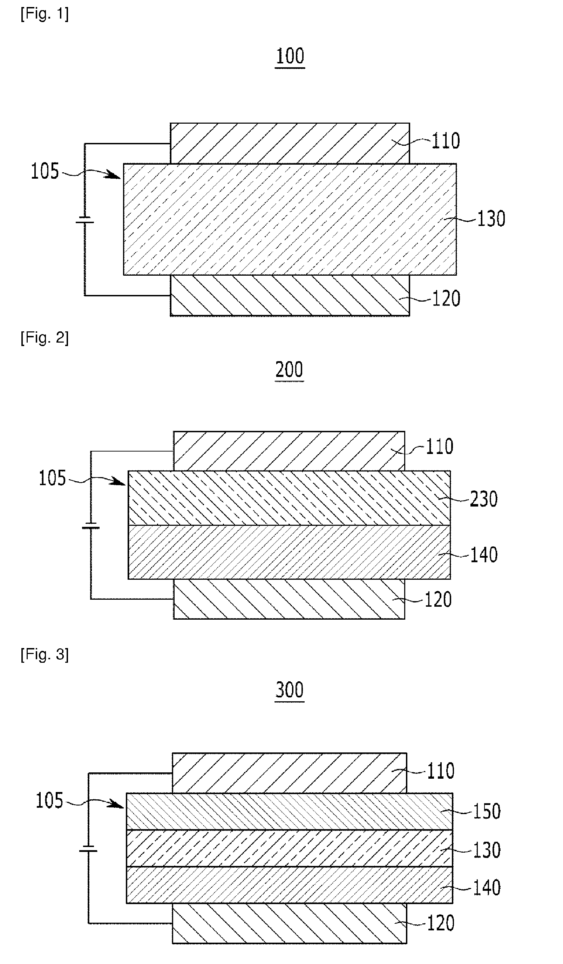

FIGS. 1 to 5 are cross-sectional views showing an organic light emitting diode including the compound for an organic optoelectronic device according to one embodiment of the present invention.

Referring to FIGS. 1 to 5, organic light emitting diodes 100, 200, 300, 400, and 500 according to one embodiment include at least one organic thin layer 105 interposed between an anode 120 and a cathode 110.

The anode 120 includes an anode material laving a large work function to help hole injection into an organic thin layer. The anode material includes: a metal such as nickel, platinum, vanadium, chromium, copper, zinc, and gold, or alloys thereof; a metal oxide such as zinc oxide, indium oxide, indium tin oxide (ITO), and indium zinc oxide (IZO); a combined metal and oxide such as ZnO:Al or SnO₂:Sb; or a conductive polymer such as poly(3-methylthiophene), poly[3,4-(ethylene-1,2-dioxy)thiophene] (PEDT), polypyrrole, and polyaniline, but is not limited thereto. In one embodiment, it is preferable to include a transparent electrode including indium tin oxide (ITO) as an anode.

The cathode 110 includes a cathode material having a small work function to help electron injection into an organic thin layer. The cathode material includes: a metal such as magnesium, calcium, sodium, potassium, titanium, indium, yttrium, lithium, gadolinium, aluminum, silver, tin, lead, cesium, barium, and the like, or alloys thereof, or a multi-layered material such as LiF/Al, LiO₂/Al, LiF/Ca, LiF/Al, and BaF₂/Ca, but is not limited thereto. In one embodiment, it is preferable to include a metal electrode including aluminum as a cathode.

Referring to FIG. 1, the organic light emitting diode 100 includes an organic thin layer 105 including only an emission layer 130.

Referring to FIG. 2, a double-layered organic light emitting diode 200 includes an organic thin layer 105 including an emission layer 230 including an electron transport layer (ETL), and a hole transport layer (HTL) 140. The emission layer 130 also functions as an electron transport layer (ETL), and the hole transport layer (HTL) 140 layer has an excellent binding property with a transparent electrode such as ITO or an excellent hole transporting property.

Referring to FIG. 3, a three-layered organic light emitting diode 300 includes an organic thin layer 105 including an electron transport layer (ETL) 150, an emission layer 130, and a hole transport layer (HTL) 140. The emission layer 130 is independently installed, and layers having an excellent electron transporting property or an excellent hole transporting property are separately stacked.

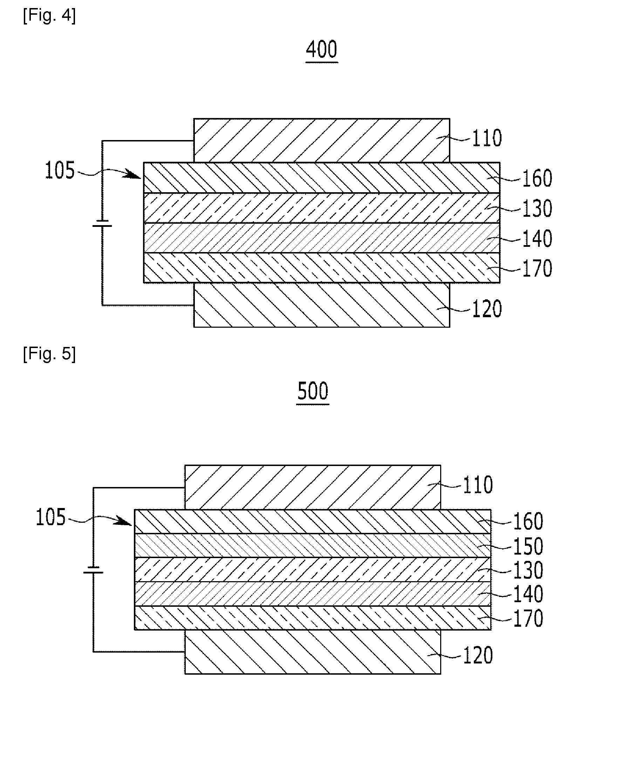

As shown in FIG. 4, a four-layered organic light emitting diode 400 includes an organic thin layer 105 including an electron injection layer (EIL) 160, an emission layer 130, a hole transport layer (HTL) 140, and a hole injection layer (HIL) 170 for binding with the cathode of ITO.

As shown in FIG. 5, a five layered organic light emitting diode 500 includes an organic thin layer 105 including an electron transport layer (ETL) 150, an emission layer 130, a hole transport layer (HTL) 140, and a hole injection layer (HIL) 170, and further includes an electron injection layer (EIL) 160 to achieve a low voltage.

In FIG. 1 to FIG. 5, the organic thin layer 105 including at least one selected from the group consisting of an electron transport layer (ETL) 150, an electron injection layer (EIL) 160, an emission layer 130 and 230, a hole transport layer (HTL) 140, a hole injection layer (HIL) 170, and combinations thereof includes the compound for an organic optoelectronic device. The compound for an organic optoelectronic device may be used for an electron transport layer (ETL) 150 including the electron transport layer (ETL) 150 or electron injection layer (EIL) 160. When it is used for the electron transport layer (ETL), it is possible to provide an organic light emitting diode having a simpler structure because it does not require an additional hole blocking layer (not shown).

Furthermore, when the compound for an organic optoelectronic device is included in the emission layers 130 and 230, the material for an organic optoelectronic device may be included as a phosphorescent or fluorescent host or a fluorescent blue dopant.

The organic light emitting diode may be fabricated by: forming an anode on a substrate; forming an organic thin layer in accordance with a dry coating method such as evaporation, sputtering, plasma plating, and ion plating, or a wet coating method such as spin coating, dipping, and flow coating; and providing a cathode thereon.

Another embodiment of the present invention provides a display device including the organic light emitting diode according to the above embodiment.

Hereinafter, the embodiments are illustrated in more detail with reference to examples. However, the following are exemplary embodiments and are not limiting.

### (Preparation of Compound for an organic optoelectronic device)

### Example 5: Compound B-1

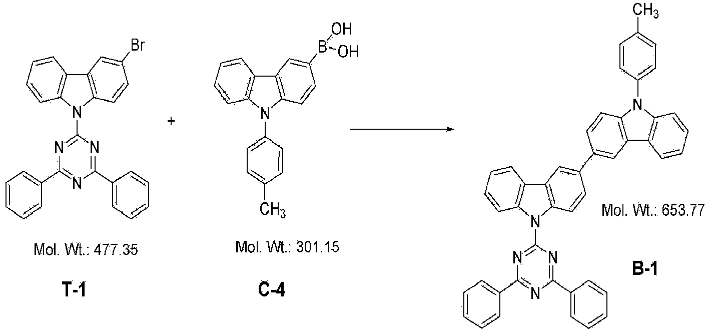

9.5g of an intermediate compound T-3 and 7.2g of an intermediate compound C-4 were dissolved in 100 mℓ of tetrahydrofuran in a 250mℓ round-bottomed flask with a thermometer, and 80 mℓ of a 2M-potassiumcarbonate aqueous solution was added thereto. Next, 1.2 g of tetrakistriphenylphosphine palladium was added to the mixture. The resulting mixture was refluxed for 12 hours. When the reaction was complete, the reactant was several times extracted with methylenechloride and treated with anhydrous sulfuric acid magnesium to remove moisture, and then, a solvent therein was removed.

The resulting reactant was purified by performing column chromatography and recrystallization, obtaining 8.5g of a compound B-1. The compound B-1 synthesized as LC-Mass was identified to have 654.74 of a [M+H]⁺ molecular weight.

### Example 6: Compound B-2

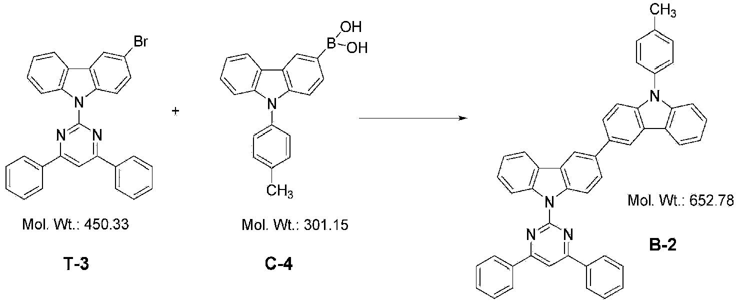

9.0g of an intermediate compound T-3 and 7.2g of an intermediate compound C-4 were dissolved in 100 mℓ of tetrahydrofuran in a 250 mℓ round-bottomed flask with a thermometer, a reflux-condenser, and an agitator under a nitrogen atmosphere, and 80 mℓ of a 2M-potassiumcarbonate aqueous solution was added thereto. Next, 1.2 g of tetrakistriphenylphosphine palladium was added to the mixture. The resulting mixture was refluxed for 12 hours. When the reaction was complete, the reactant was several times extracted with methylenechloride, treated with anhydrous sulfuric acid magnesium to remove moisture, and filtrated, and then, a solvent therein was removed.

The resulting reactant was purified by performing column chromatography and recrystallization, obtaining 9.2g of a compound B-2. The compound B-2 synthesized as LC-Mass was identified to have 654.74 of a [M+H]⁺ molecular weight.

### Comparative Example 1: Compound R-1

A compound represented by the following Chemical Formula R-1 was prepared.

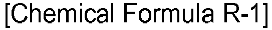

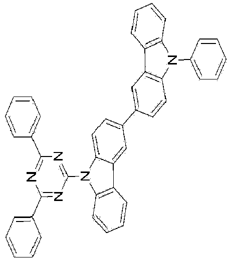

### Comparative Example 2: Compound R-2

A compound represented by the following Chemical Formula R-2 was prepared.

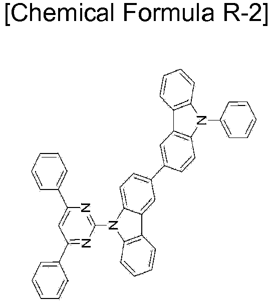

### Comparative Example 3: Compound R-3

A compound represented by the following Chemical Formula R-3 was prepared.

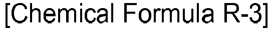

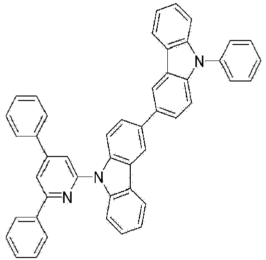

### (Result of Energy Level Calculation)

The compounds synthesized according to Examples 5 and 6 and Comparative Examples 1 to 3 were calculated regarding energy level with Gaussian 03 (b3lyp/6-31g). The results are provided in Table 1.

**(Table 1)**

| Material | ETU | R | HOMO (eV) | LUMO (eV) | ΔE (T1) | ΔE (S1) |
|---|---|---|---|---|---|---|
| Comparative Example 1 (R-1) | triazinyl group | hydrogen | -5.16 | -1.89 | 2.83 | 2.90 |
| Example 5 (B-1) | triazinyl group | p-methyl | -5.12 | -1.88 | 2.82 | 2.88 |
| Comparative | pyrimidinyl | hydrogen | -5.05 | -1.72 | 2.78 | 2.93 |
| Example 2 (R-2) | group | | | | | |
| Example 6 (B-2) | pyrimidinyl group | p-methyl | -5.00 | -1.76 | 2.71 | 2.84 |
| Comparative Example 3 (R-3) | pyridinyl group | hydrogen | -4.97 | -1.45 | 2.91 | 3.09 |

Referring to Table 1, ETU indicates ETU represented by the above Chemical Formula 1, and the R indicates at least substituent among R¹ to R⁵ in the above Chemical Formula 1.

As a result, the compound according to Examples had different energy level characteristic depending on ETU of the materials but not on kinds of R and location of a substituent.

### (Preparation of Organic light emitting diode)

### Example 11: Preparation of Organic Light Emitting Diode

A glass substrate having a 1500Å-thick ITO (Indium tin oxide) layer was cleaned with ultrasonic waves using distilled water. Next, the resulting substrate was cleaned with ultrasonic waves using a solvent such as isopropyl alcohol, acetone, methanol, and the like and dried. The dried substrate was moved to a plasma cleaner and cleaned by using an oxygen plasma for 5 minutes there and then, moved to a vacuum depositor. The ITO transparent electrode was used as an anode, and the following HTM compound was vacuum-deposited to form a 1200Å-thick hole injection layer (HIL).

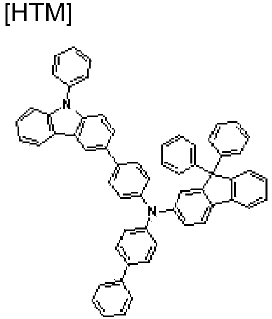

On the hole transport layer (HTL), a 300Å-thick emission layer was formed by doping the material synthesized in Example 5 as a host with 7wt% of the following PhGD compound as a green phosphorescent dopant and vacuum-depositing the doped material.

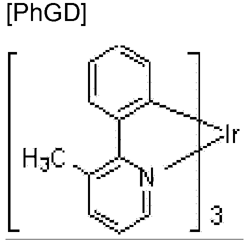

Next, electron transport layer (ETL) was formed on the emission layer by laminating the following BAlq [bis(2-methyl-8-quinolinolato-N1,O8)-(1,1'-biphenyl-4-olato)aluminum] compound to be 50Å thick and sequentially, the following Alq3 [tris (8-hydroxyquinolinato)aluminium] compound to be 250Å thick thereon. Then, 5Å-thick LiF and 1000Å-thick Al were sequentially vacuum-deposited on the electron transport layer (ETL), fabricating a cathode and thus, an organic light emitting diode.

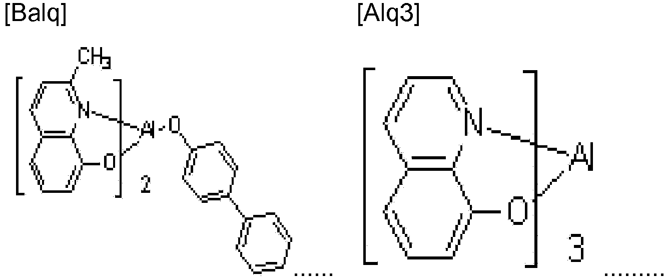

### Example 12

An organic light emitting diode was fabricated according to the same method as Example 11 except for using the compound of Example 6 instead of the compound of Example 5.

### Comparative Example 4

An organic light emitting diode was fabricated according to the same method as Example 11 except for using the compound of Comparative Example 1 instead of the compound of Example 5 as a host.

### Comparative Example 5

An organic light emitting diode was fabricated according to the same method as Example 11 except for using the compound of Comparative Example 2 instead of the compound of Example 5 as a host.

### Comparative Example 6

An organic light emitting diode was fabricated according to the same method as Example 11 except for using the compound of Comparative Example 3 instead of the compound of Example 5 as a host.

### (Performance Evaluation of Organic light emitting diode)

Each organic light emitting diode according to Examples 11 and 12 and Comparative Examples 4 to 6 was measured regarding current density change, luminance change, and luminous efficiency depending on a voltage.

### (1) Current density change depending on Voltage change

The organic light emitting diodes were measured regarding current by increasing a voltage from 0 V to 10 V with a current-voltage meter (Keithley 2400). The current was divided with an area.

### (2) Luminance change depending on Voltage change

The organic light emitting diodes were measured regarding luminance by increasing a voltage from 0 V to 10 V with a luminance meter (Minolta Cs-1000A).

### (3) Luminous efficiency measurement

The luminance and current density obtained in the above (1) and (2) and a voltage were used to calculate current efficiency (cd/A) at the same current density (10 mA/cm²).

### (4) Device life-span measurement

Measured was time it took for luminance to drop from 3000cd/m² to 2910cd/m² by 3%.

The following Table 2 shows the device evaluation result of a compound including a triazinyl group as ETU in the above Chemical Formula 1.

**(Table 2)**

| | Host | Vd | Cd/A | Im/W | cd/m² | CIEx | CIEy | Life-span (h) |
|---|---|---|---|---|---|---|---|---|
| Comparative Example 4 | R-1 | 5.21 | 53.5 | 32.3 | 3000 | 0.338 | 0.623 | 10 |

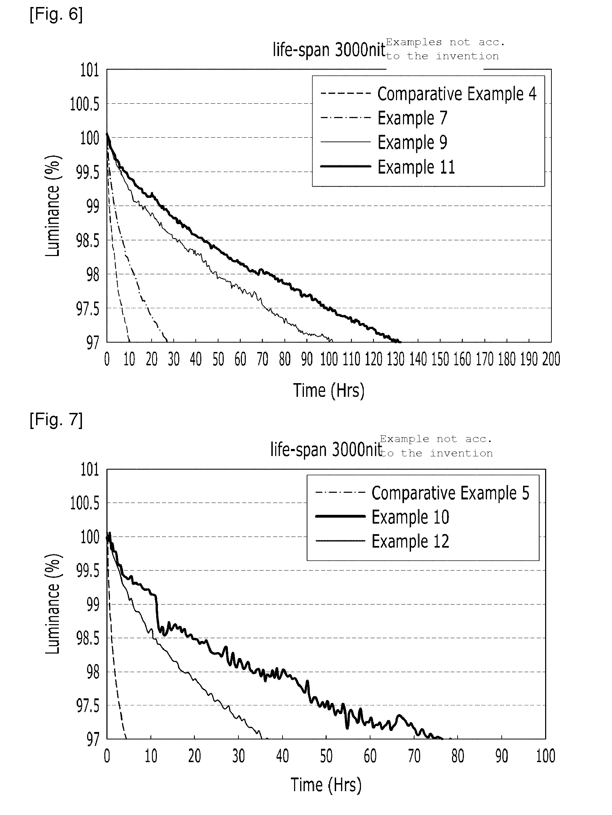

FIG. 6 shows life-span data of the light emitting diodes according to Example 11 and Comparative Example 4. The light emitting diodes including an emission layer prepared by applying B-1 according to Examples as a host had improved efficiency and life-span compared with the light emitting diodes an emission layer prepared by applying R-1 according to Comparative Example 1 as a host.

The following Table 3 shows the evaluation results of the devices including a compound having a pyrimidinyl group as ETU in the above Chemical Formula 1.

**(Table 3)**

| | Host | Vd | Cd/A | ImNV | cd/m² | CIEx | CIEy | Life-span (h) |
|---|---|---|---|---|---|---|---|---|
| Comparative Example 5 | R-2 | 5.04 | 58.4 | 36.4 | 3000 | 0.341 | 0.621 | 4 |
| Example 12 | B-2 | 4.91 | 66.3 | 42.5 | 3000 | 0.339 | 0.623 | 37 |

FIG. 7 shows life-span data of the organic light emitting diodes according to Example 12 and Comparative Example 5.

The organic light emitting diode including an emission layer using B-2 as a host according to Examples had improved efficiency and life-span compared with the organic light emitting diode including an emission layer using R-2 as a host according to Comparative Example 2.

The following Table 4 show the devices evaluation results of the devices including a compound having a pyridinyl group as ETU in the above Chemical Formula 1.

**(Table 4)**

| | Host | Vd | Cd/A | Im/W | cd/m² | CIEx | CIEy | Life-span (h) |
|---|---|---|---|---|---|---|---|---|
| Comparative Example 6 | R-3 | 5.14 | 58.4 | 35.7 | 3000 | 0.335 | 0.624 | 20 |

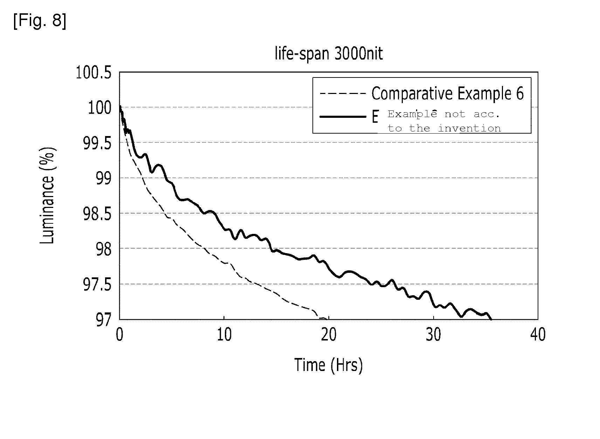

FIG. 8 shows life-span data of an organic light emitting diode not according to the invention and that of Comparative Example 6.

Based on the energy level characteristic evaluation in Table 1 and the device evaluation in Tables 2 to 4, when an alkyl group other than hydrogen was added to a phenyl group substituted for bicarbazole in a derivative with similar energy level characteristic, a device had improved performance and particularly, increased life-span.

### <Description of symbols>

| | | | |
|---|---|---|---|
| 100 : | organic light emitting diode | 110 : | cathode |
| 120 : | anode | 105 : | organic thin layer |
| 130 : | emission layer | 140 : | hole transport layer (HTL) |
| 150 : | electron transport layer (ETL) | 160 : | electron injection layer (EIL) |
| 170 : | hole injection layer (HIL) | 230 : | emission layer + electron transport layer (ETL) |

## Claims

1. A compound for an organic optoelectronic device **characterized in, that** the compound has the following Chemical Formula 1: 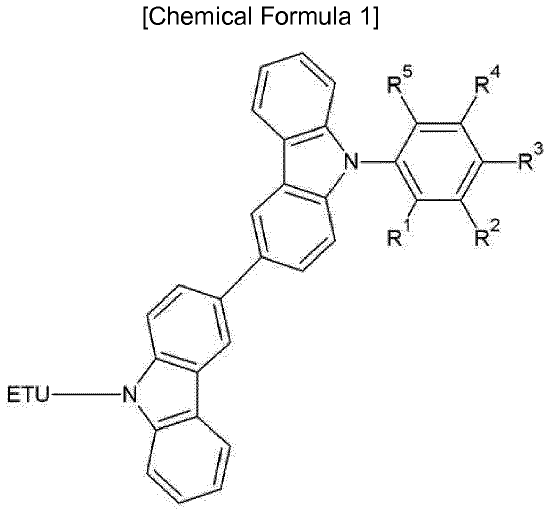 wherein, in Chemical Formula 1,
ETU is a substituted or unsubstituted pyridinyl group, a substituted or unsubstituted pyrimidinyl group, a substituted or unsubstituted triazinyl group, or a combination thereof, and
R¹ to R⁵ are the same or different and independently hydrogen, deuterium, a substituted or unsubstituted C1 to C30 alkyl group, or a combination thereof,
**characterized in that**
at least one of R¹ to R⁵ is a substituted or unsubstituted. C1 to C30 alkyl group.

2. The compound for an organic optoelectronic device of claim 1, wherein at least one of R² to R⁴ is a substituted or unsubstituted methyl group.

3. The compound for an organic optoelectronic device of claim 1 or 2, wherein the ETU is a substituent represented by the following Chemical Formulas 2 to 6:

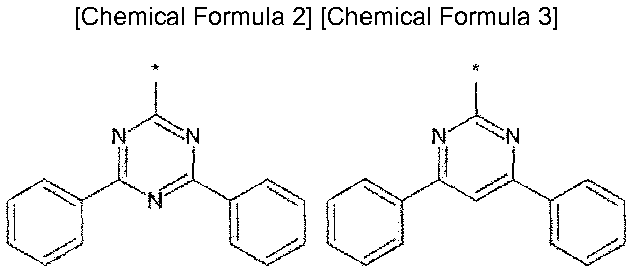

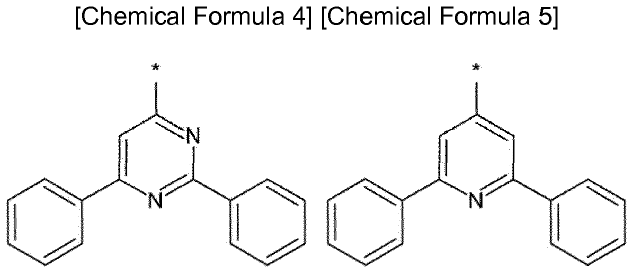

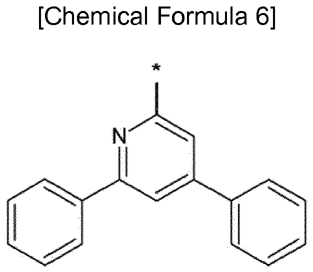

4. The compound for an organic optoelectronic device of claims 1 to 3, wherein the compound for an organic optoelectronic device is represented by one of the following Chemical Formulae B-1 to B-13, and B-19 to B-25:

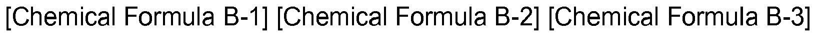

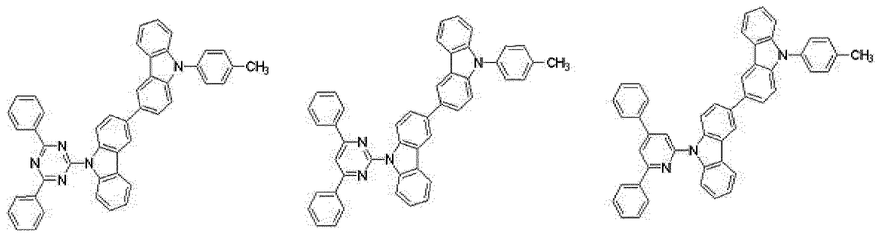

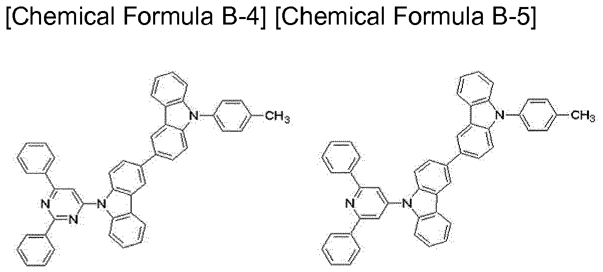

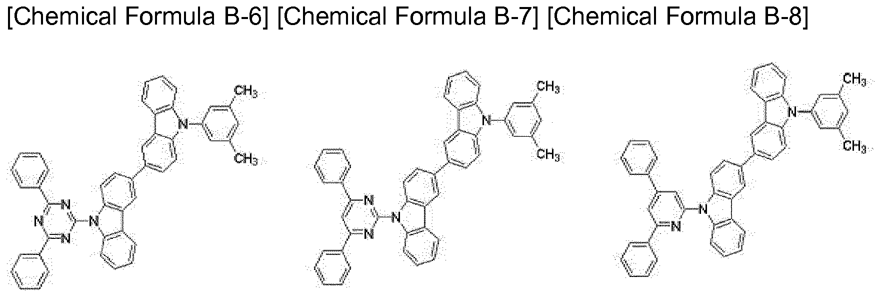

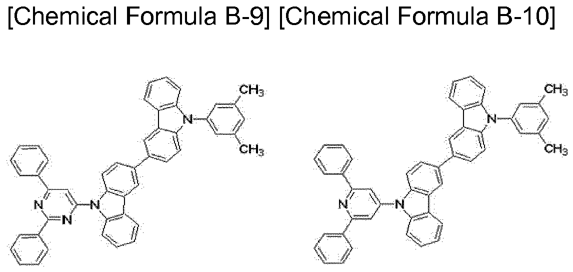

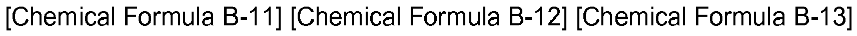

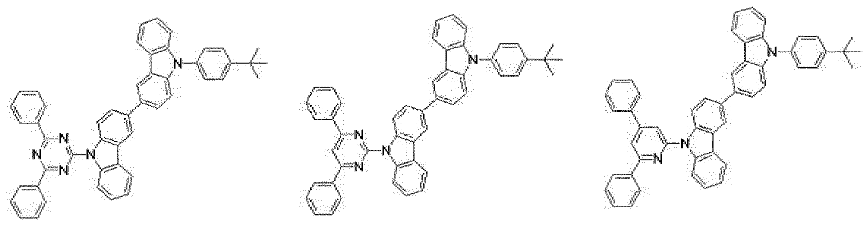

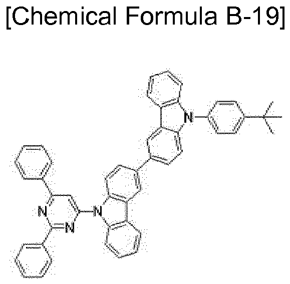

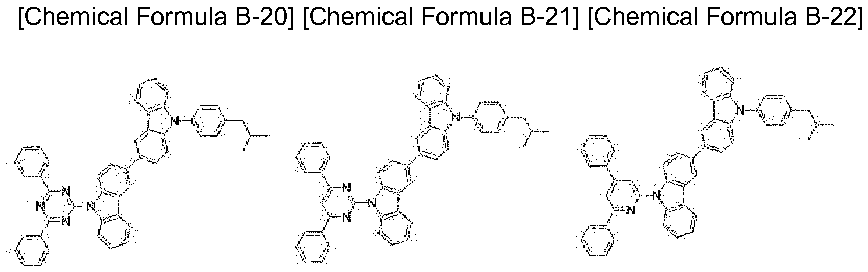

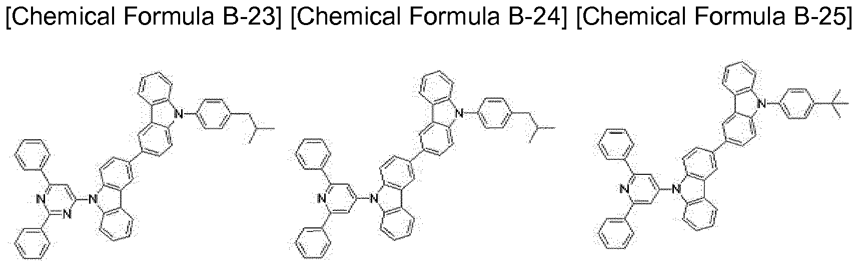

5. The compound for an organic optoelectronic device of claims 1 to 4, wherein the compound for an organic optoelectronic device has a triplet excitation energy (T1) of 2.0eV or more.

6. The compound for an organic optoelectronic device of claims 1 to 5, wherein the organic optoelectronic device is selected from an organic photoelectric device, an organic light emitting diode, an organic solar cell, an organic transistor, an organic photo-conductor drum, and an organic memory device.

7. An organic light emitting diode comprising:
an anode, a cathode, and at least one or more organic thin layer between the anode and the cathode,
wherein at least one of the organic thin layer includes the compound for an organic optoelectronic device according to claims 1 to 6.

8. The organic light emitting diode of claim 7, wherein the organic thin layer is selected from an emission layer, a hole transport layer (HTL), a hole injection layer (HIL), an electron transport layer (ETL), an electron injection layer (EIL), a hole blocking layer, and a combination thereof.

9. The organic light emitting diode of claim 7 or 8, wherein the compound for an organic optoelectronic device is included in a hole transport layer (HTL) or a hole injection layer (HIL).

10. The organic light emitting diode of claims 7 to 9, wherein the compound for an organic optoelectronic device is included in an emission layer.

11. The organic light emitting diode of claims 7 to 10, wherein the compound for an organic optoelectronic device is used as a phosphorescent or fluorescent host material in an emission layer.

12. A display device including the organic light emitting diode, comprising the organic light emitting diode according to claims 7 to 11.

## Patentansprüche

1. Verbindung für eine organische optoelektronische Vorrichtung, **dadurch gekennzeichnet, dass** die Verbindung die folgende Chemische Formel 1 aufweist: 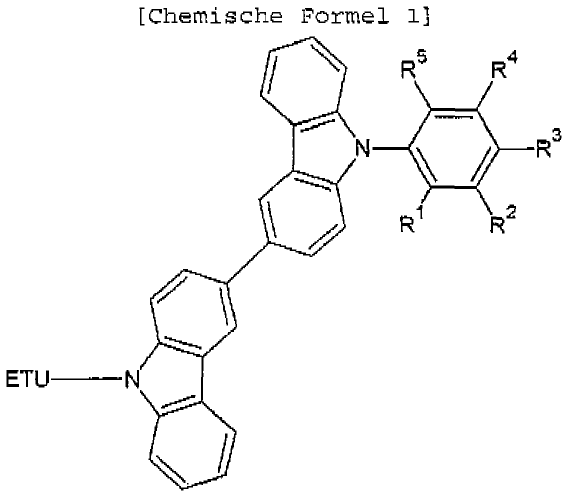 wobei in der Chemischen Formel 1
ETU für eine substituierte oder unsubstituierte Pyridinylgruppe, eine substituierte oder unsubstituierte Pyrimidinylgruppe, eine substituierte oder unsubstituierte Triazinylgruppe oder eine Kombination davon steht und
R¹ bis R⁵ gleich oder verschieden sind und unabhängig für Wasserstoff, Deuterium, eine substituierte oder unsubstituierte C1- bis C30-Alkylgruppe oder eine Kombination davon stehen,
**dadurch gekennzeichnet, dass**
mindestens eine der Variablen R¹ bis R⁵ für eine substituierte oder unsubstituierte C1- bis C30-Alkylgruppe steht.

2. Verbindung für eine organische optoelektronische Vorrichtung nach Anspruch 1, wobei mindestens eine der Variablen R² bis R⁴ für eine substituierte oder unsubstituierte Methylgruppe steht.

3. Verbindung für eine organische optoelektronische Vorrichtung nach Anspruch 1 oder 2, wobei es sich bei dem ETU um einen Substituenten handelt, der durch die folgenden Chemischen Formeln 2 bis 6 wiedergegeben wird:

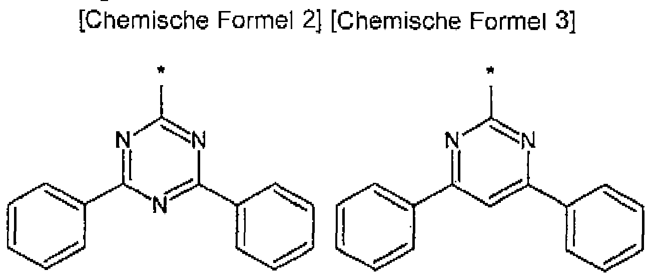

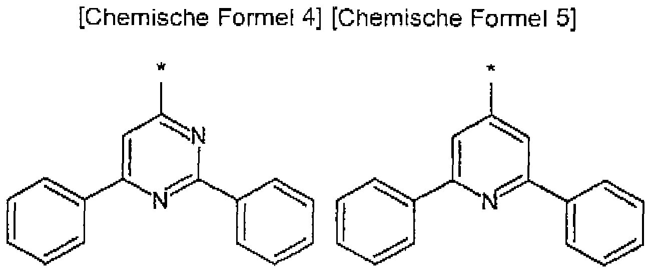

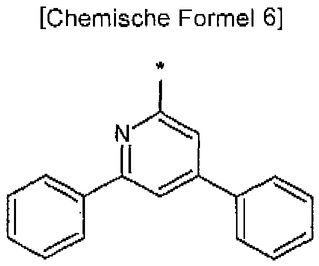

4. Verbindung für eine organische optoelektronische Vorrichtung nach den Ansprüchen 1 bis 3, wobei die Verbindung für eine organische optoelektronische Vorrichtung durch eine der folgenden Chemischen Formeln B-1 bis B-13 und B-19 bis B-25 wiedergegeben wird:

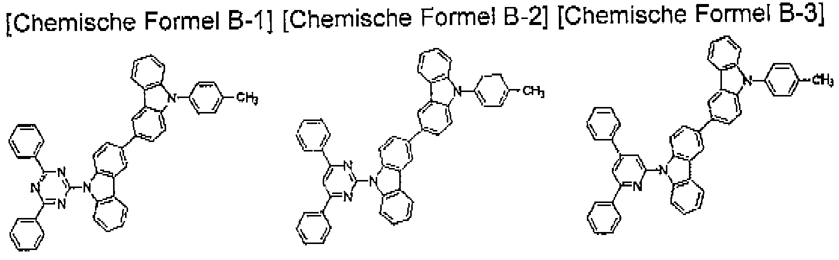

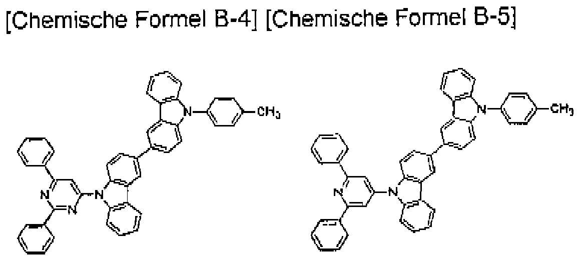

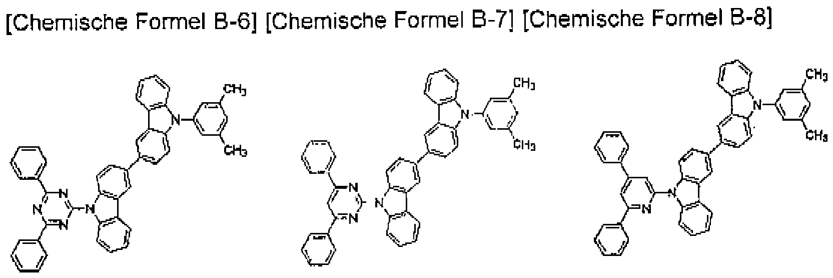

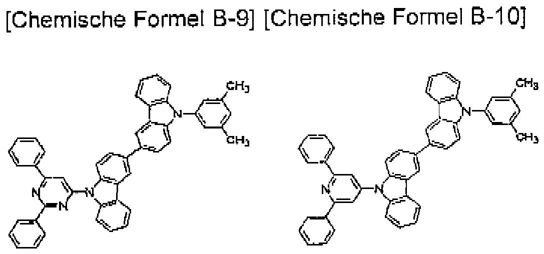

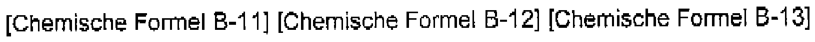

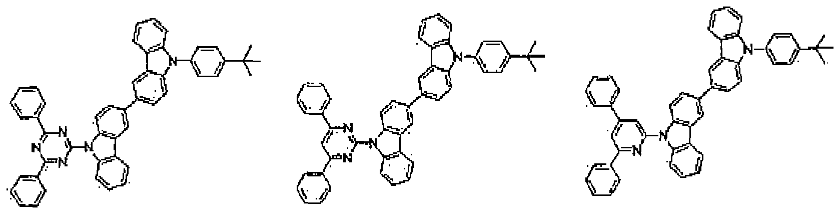

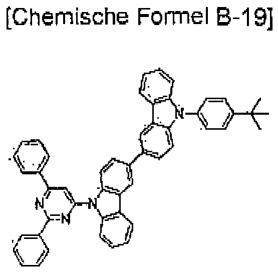

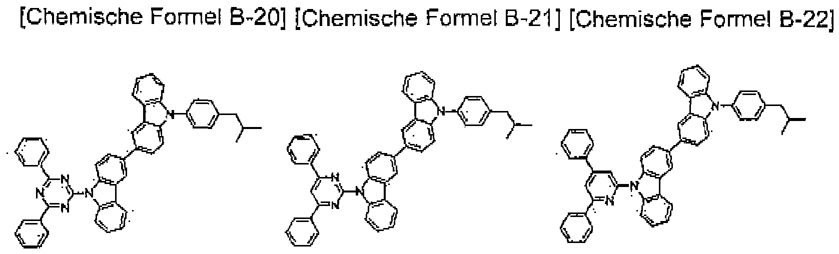

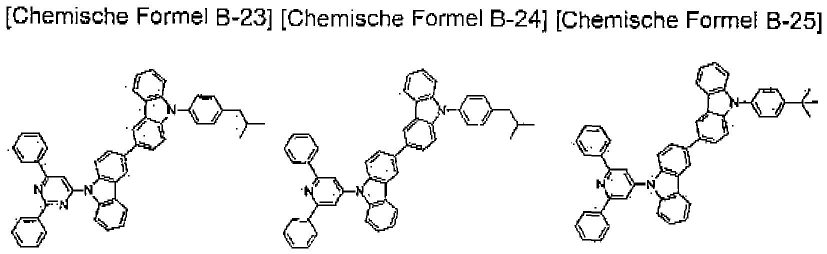

5. Verbindung für eine organische optoelektronische Vorrichtung nach den Ansprüchen 1 bis 4, wobei die Verbindung für eine organische optoelektronische Vorrichtung eine Triplett-Anregungsenergie (T1) von 2,0 eV oder mehr aufweist.

6. Verbindung für eine organische optoelektronische Vorrichtung nach den Ansprüchen 1 bis 5, wobei die organische optoelektronische Vorrichtung aus einer organischen photoelektrischen Vorrichtung, einer organischen Leuchtdiode, einer organischen Solarzelle, einem organischen Transistor, einer organischen Photoleitertrommel und einer organischen Speichervorrichtung ausgewählt ist.

7. Organische Leuchtdiode, umfassend:
eine Anode, eine Kathode und mindestens eine oder mehrere organische dünne Schichten zwischen der Anode und der Kathode ,
wobei mindestens eine der organischen dünnen Schichten die Verbindung für eine organische optoelektronische Vorrichtung nach den Ansprüchen 1 bis 6 enthält.

8. Organische Leuchtdiode nach Anspruch 7, wobei die organische dünne Schicht aus einer Emissionsschicht, einer Lochtransportschicht (Hole Transport Layer, HTL), einer Lochinjektionsschicht (Hole Injektion Layer, HIL), einer Elektrodentransportschicht (Electron Transport Layer, ETL), einer Elektroneninjektionsschicht (Electron Injection Layer, EIL), einer Lochblockierschicht und einer Kombination davon ausgewählt ist.

9. Organische Leuchtdiode nach Anspruch 7 oder 8, wobei die Verbindung für eine organische optoelektronische Vorrichtung in einer Lochtransportschicht (HTL), oder einer Lochinjektionsschicht (HIL) enthalten ist.

10. Organische Leuchtdiode nach den Ansprüchen 7 bis 9, wobei die Verbindung für eine organische optoelektronische Vorrichtung in einer Emissionsschicht enthalten ist.

11. Organische Leuchtdiode nach den Ansprüchen 7 bis 10, wobei die Verbindung für eine organische optoelektronische Vorrichtung als phosphoreszierendes oder fluoreszierendes Wirtsmaterial in einer Emissionsschicht verwendet wird.

12. Anzeigevorrichtung mit der organischen Leuchtdiode, umfassend die organische Leuchtdiode nach den Ansprüchen 7 bis 11.

## Revendications

1. Composé pour un dispositif optoélectronique organique **caractérisé en ce que** le composé à la formule chimique 1 suivante : 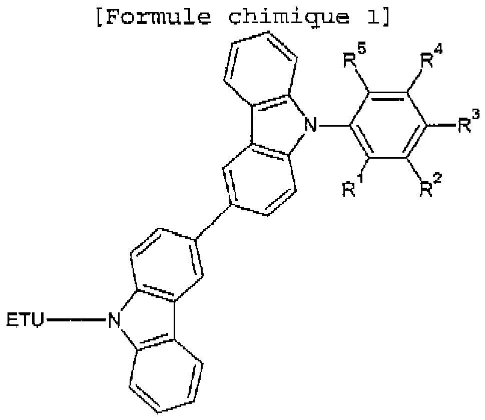 dans lequel, dans la formule chimique 1,
ETU est un groupe pyridinyle substitué ou non substitué, un groupe pyrimidinyle substitué ou non substitué, un groupe triazinyle substitué ou non substitué, ou une combinaison de ceux-ci, et
les R¹ à R⁵ sont identiques ou différents et représentent indépendamment un hydrogène, un deutérium, un groupe alkyle en C1 à C30 substitué ou non substitué, ou une combinaison de ceux-ci,
**caractérisé en ce que**
au moins un des R¹ à R⁵ est un groupe alkyle en C1 à C30 substitué ou non substitué.

2. Composé pour un dispositif optoélectronique organique de la revendication 1, dans lequel au moins un des R² à R⁴ est un groupe méthyle substitué ou non substitué .

3. Composé pour un dispositif optoélectronique organique de la revendication 1 ou 2, dans lequel l'ETU est un substituant représenté par les formules chimiques 2 à 6 suivantes :

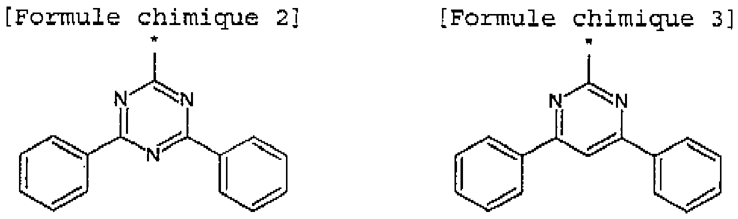

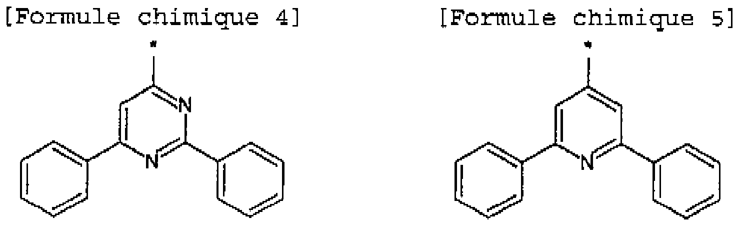

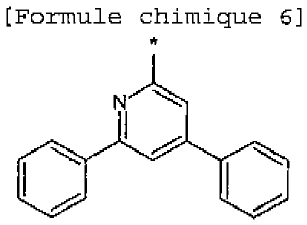

4. Composé pour un dispositif optoélectronique organique des revendications 1 à 3, le composé pour un dispositif optoélectronique organique étant représenté par une des formules chimiques B-1 à B-13 et B-19 à B-25 suivantes :

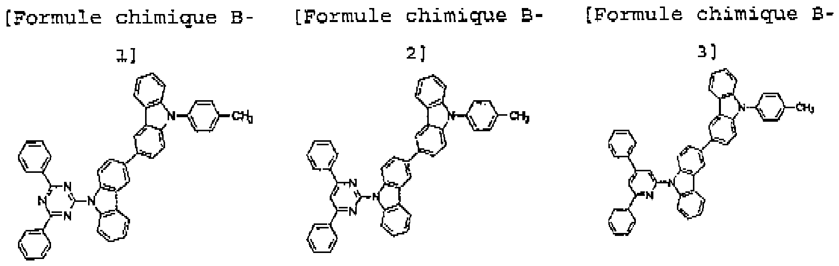

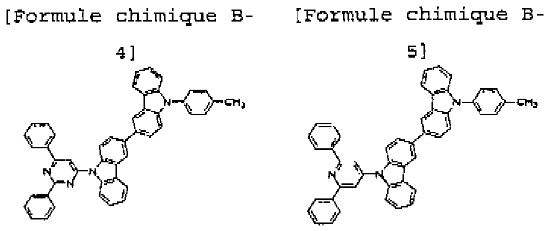

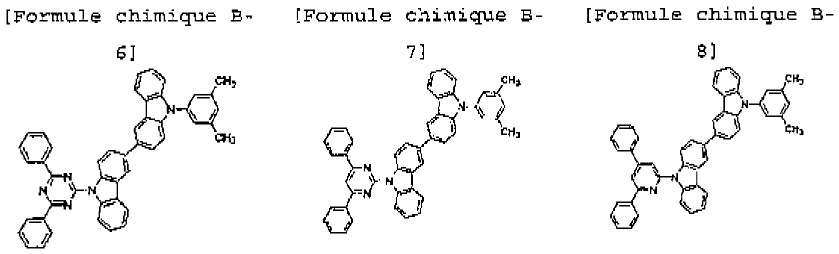

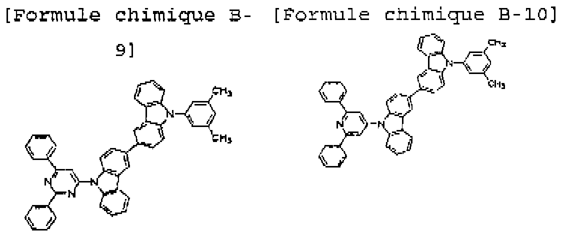

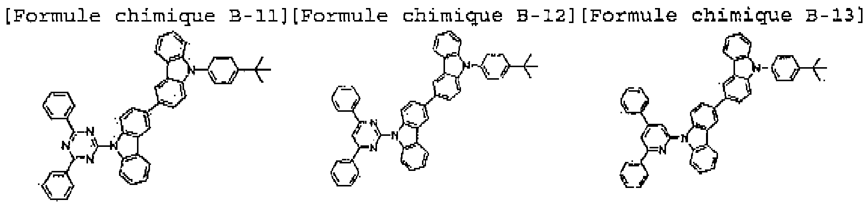

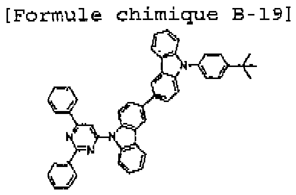

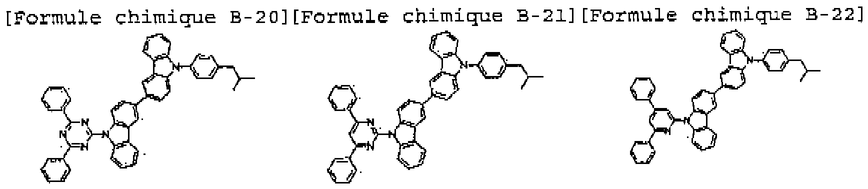

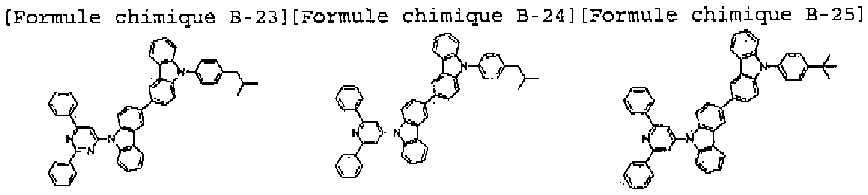

5. Composé pour un dispositif optoélectronique organique des revendications 1 à 4, le composé pour un dispositif optoélectronique organique ayant une énergie d'excitation de triplet (T1) d'au moins 2,0 eV.

6. Composé pour un dispositif optoélectronique organique des revendications 1 à 5, le dispositif optoélectronique organique étant choisi parmi un dispositif photoélectrique organique, une diode électroluminescente organique, une cellule salaire organique, un transistor organique, un tambour photoconducteur organique, et un dispositif de mémoire organique.

7. Diode électroluminescente organique comprenant :
une anode, une cathode, et au moins une ou plusieurs couches minces organiques entre l'anode et la cathode,
au moins une des couches minces organiques contenant le composé pour un dispositif optoélectronique organique selon les revendications 1 à 6.

8. Diode électroluminescente organique de la revendication 7, dans laquelle la couche mince organique est choisie parmi une couche d'émission, une couche de transport de trous (HTL), une couche d'injection de trous (HIL), une couche de transport d'électrons (ETL), une couche d'injection d'électrons (EIL), une couche de blocage de trous, et une combinaison de celles-ci.

9. Diode électroluminescente organique de la revendication 7 ou 8, le composé pour un dispositif optoélectronique organique étant contenu dans une couche de transport de trous (HTL) ou une couche d'injection de trous (HIL).

10. Diode électroluminescente organique des revendications 7 à 9, le composé pour un dispositif optoélectronique organique étant contenu dans une couche d'émission.

11. Diode électroluminescente organique des revendications 7 à 10, le composé pour un dispositif optoélectronique organique étant utilisé comme matériau hôte phosphorescent ou fluorescent dans une couche d'émission.

12. Dispositif d'affichage comportant la diode électroluminescente organique, comprenant la diode électroluminescente organique selon les revendications 7 à 11.
